# DEMANDE DE BREVET EUROPEEN

(11) **EP 2 202 293 A2**
(43) Date de publication de la demande: **30.06.2010**
(21) Numéro de dépôt: 10155035.8
(22) Date de dépôt: 25.05.2005
(51) Int. Cl.: C12N 1/20, C12P 17/02, A61K 31/357, A61P 31/04, A61P 35/00, A61P 31/12

(54) **Nouveaux composés actifs mutactimycines et aldgamycines et leur utilisation thérapeutique**

(30) Priorité: 26.05.2004 FR 0405659
(62) Demande divisionnaire de: 05773198.6
(71) Demandeur: Institute National Polytechnique de Toulouse, 31000 Toulouse (FR)
(72) Inventeur: Zitouni, Abdelghani, 16300 Alger (DZ); Sabaou, Nasserdine, 16300 Alger (DZ); Mathieu, Florence, 31750 Escalquens (FR); Lebrihi, Ahmed, 31450 Noueilles (FR)
(74) Mandataire: Warcoin, Jacques

(57) **Abrégé**

Un des objets de l'invention est la mutactimycine G de formule suivante : 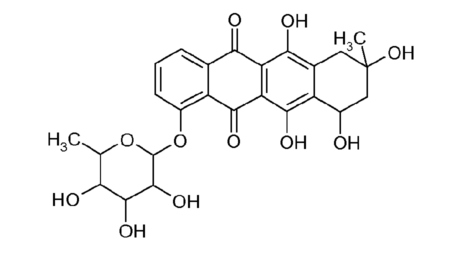 ou ses sels d'addition pharmaceutiquement acceptables, isomères, énantiomères, diastéréoismères, ainsi que leurs mélanges.

L'invention porte également sur la mutactimycine PR, la mutactimycine F, l'aldgamycine G, l'aldgamycine H et l'aldgamycine P10b, ainsi que leur utilisation en tant que médicament.

## Description

L'invention a pour objet une nouvelle souche actinomycète *Saccharothrix* SA 103 déposée à la CNCM le 6 février 2004 sous le numéro I-3160 ou une souche mutante de celle-ci, un procédé et un milieu de sélection de cette souche, un procédé de production d'un bouillon de culture, d'un concentrat actif et de composés actifs à partir d'une culture de cette souche SA 103. L'invention a également pour objet les composés actifs susceptibles d'être obtenues par le procédé de production, notamment de nouvelles mutactimycines et aldgamycines, des compositions pharmaceutiques comprenant ces composés actifs et leur utilisation dans le domaine médical et phytopharmaceutique.

Les agents antimicrobiens sont largement utilisés dans de nombreux domaines : santé humaine, vétérinaire, phytopathologie, industrie alimentaire, traitement du cuir et du bois, etc. La plupart de ces agents sont produits par des microorganismes, dont les actinomycètes sont parmi les plus importants.

On cherche toujours de nouveaux agents antimicrobiens, en particulier des antibiotiques antibactériens.

En effet, on sait que de par la nature même des bactéries, celles-ci prolifèrent de manière exponentielle, et augmentent ainsi leurs chances de devenir résistantes à des antibiotiques, ceci suite à des mutations génétiques.

L'utilisation inadéquate d'antibiotiques dans le domaine animal, notamment l'homme, ou végétal, mais aussi dans l'industrie alimentaire ou l'industrie du cuir, accroît la capacité mutationnelle des bactéries, et donc leur résistance à des antibiotiques.

Une solution à ce problème est bien sûr d'administrer à bon escient les antibiotiques, mais cette solution ne peut être que préventive.

Il existe donc un besoin, dans l'état actuel de la technique, de développer de nouveaux antibiotiques. Pour ce faire, les recherches se sont portées sur les microorganismes extrêmophiles.

Ainsi, les inventeurs ont isolé une nouvelle souche actinomycète *Saccharothrix* SA 103 présente dans le sol sud saharien de l'Algérie, et l'ont déposée à la Collection Nationale de Cultures de Microorgansimes (CNCM) le 16 février 2004 sous le numéro I-3160.

Ils ont également découvert que cette souche produisait des composés actifs de la classe des anthracyclines, dont certains n'ont jamais été identifiés auparavant, notamment des mutactimycines (PR, F, G), mais également des composés actifs de la classe des anthracyclines, notamment des aldgamycines (G, H et P10b). Ces composés actifs présentent une activité antibactérienne, en particulier contre les bactéries Gram-positives, mais également une activité antivirale, antiproliférative et anticancéreuse.

Ils ont dès lors mis au point un procédé d'obtention de ces composés à partir de la souche *Saccharothrix* SA 103 déposée à la CNCM le 16 février 2004 sous le numéro I-3160, lequel procédé présente l'avantage d'être simple à mettre en oeuvre. Ainsi, ces composés antibiotiques obtenus à partir de la souche *Saccharothrix* présentent un réel intérêt pharmaceutique, aussi bien pour l'homme que pour l'animal, ainsi qu'un intérêt phytosanitaire, et peuvent être produits à l'échelle industrielle.

Ceci est justement l'objet de la présente invention.

Ainsi, l'invention a tout d'abord pour objet la souche actinomycète *Saccharothrix* SA 103 déposée à la CNCM le 16 février 2004 sous le numéro I-3160 ou une souche mutante de celle-ci.

Par « souche mutante », « mutant », « souche variante » ou « variant », on entend désigner de manière indifférente dans la présente demande une souche de *Saccharothrix* pouvant être obtenue par mutation sélective à partir de la souche *Saccharothrix* SA 103 en conservant la capacité de produire au moins l'un des composés actifs décrits ci-après. Les techniques de mutation sont connues de l'homme du métier et consistent à mettre la souche *Saccharothrix* SA 103 en présence d'un agent mutagène physique, tel qu'un rayonnement, ou d'un agent mutagène chimique, par exemple l'acriflavine, puis à sélectionner dans un milieu approprié les mutants d'intérêt restants en utilisant leur spectre antiobiotique (méthode d'inhibition de microorganismes telle que par exemple la méthode de la concentration minimale inhibitrice ou CMI, etc...).

Les caractéristiques de la souche *Saccharothrix* SA 103 selon l'invention sont décrites dans la partie « Exemples » de la présente demande.

La souche *Saccharothrix* SA 103 selon l'invention ou l'un de ses mutants est mise en culture dans un milieu contenant une variété de substances nutritives généralement utilisées pour la croissance des actinomycètes. Par exemple, comme source de carbone, on peut utiliser du glucose, de la glycérine, du sucrose, de l'amidon, du maltose, ou bien des huiles animales ou végétales. Comme source d'azote on peut utiliser, par exemple, de l'azote organique tel que de la farine de soja, des extraits de viande, un extrait de levure, une peptone, de l'eau de macération du maïs, du tourteau de coton ou de la farine de poisson. On peut utiliser de l'azote inorganique tel que par exemple du sulfate d'ammonium, du chlorure d'ammonium, du nitrate de sodium ou du phosphate d'ammonium. Si cela est nécessaire, on peut encore ajouter du chlorure de sodium, du chlorure de potassium, du phosphate de potassium ou des sels métalliques bivalents tels que Mg⁺⁺, Ca⁺⁺ Zn⁺⁺, Fe⁺⁺, Cu⁺⁺, Mn⁺⁺ ou Ni⁺⁺, et des acides aminés ou des vitamines. On peut utiliser des géloses inclinées.

Un autre objet de la présente invention est un procédé de sélection de la souche actinomycète *Saccharothrix* SA 103 selon l'invention et/ou d'au moins l'une de ses souches mutantes, **caractérisé en ce qu**'il comprend les étapes suivantes :
a) mise en présence d'un échantillon biologique susceptible de contenir ladite souche et/ou au moins l'une de ses souches mutantes avec un milieu de sélection approprié;
b) isolement de ladite souche et/ou d'au moins l'une de ses souches mutantes.

Un autre objet de la présente invention est un milieu de sélection approprié permettant d'isoler la souche actinomycète SA 103 selon l'invention et/ou au moins l'une de ses souches mutantes.

On entend désigner par milieu de sélection approprié dans la présente demande tout milieu au moyen duquel au moins la souche actinomycète *Saccharothrix* SA 103 selon l'invention est capable de se développer mais qui empêche la croissance d'au moins une souche différente de la souche *Saccharothrix* SA 103 ou d'un autre micro-organisme tel qu'un champignon ; de préférence, le milieu de sélection selon l'invention permet d'empêcher la croissance de toutes les souches différentes de la souche *Saccharothrix* SA 103 selon l'invention ou d'une souche mutante de celle-ci.

Un tel milieu de sélection comprend une variété de substances nutritives telles que définies précédemment et au moins un produit sélectif tel que par exemple un agent antibactérien auquel seule la souche SA 103 selon l'invention et/ou au moins l'une de ses souches mutantes est résistante, et/ou un agent antifungique pour éliminer les champignons dont la croissance rapide pourrait gêner ou même empêcher la souche selon l'invention de croître.

Comme exemple de milieu de sélection approprié pour isoler la souche actinomycète SA 103 selon l'invention on peut citer le milieu M1S qui correspond à une gélose humique-vitamines B comprenant également du lysozyme, de préférence à hauteur d'environ 0,005 %, de la cyclosérine, de préférence à hauteur d'environ 10 mg/l, le pH de ce milieu étant compris dans la gamme d'environ 8,5 à environ 9.

Un autre exemple préféré de milieu de sélection approprié pour isoler la souche actinomycète SA 103 selon l'invention est le milieu HV-Vitamine B comprenant également du lysozyme, de préférence à hauteur d'environ 0,005 %, de la cyclosérine, de préférence à hauteur d'environ 10 mg/l, de la pénicilline, de préférence à hauteur d'environ 10 mg/l, ainsi que de la rifampicine, de préférence à hauteur d'environ 5 mg/l, le pH de ce milieu étant compris dans la gamme d'environ 8,5 à environ 9.

La sélectivité du milieu de sélection approprié selon l'invention peut être renforcée en ajoutant audit milieu d'autres produits sélectifs auxquels la souche SA 103 selon l'invention est résistante, tels que notamment l'azide de sodium, de préférence à hauteur d'environ 0.001 %, le tellurite de potassium, de préférence à hauteur d'environ 0.01%, et/ou le crystal violet, de préférence à hauteur d'environ 0.001 %.

Comme exemple d'agent antifungique qui peut être ajouté au milieu de sélection approprié selon l'invention, on peut citer l'actidione et/ou la nystatine.

Lorsque l'on souhaite sélectionner une souche mutante de la souche *Saccharothrix* SA 103 selon l'invention, le milieu sélectif peut également consister en un milieu de culture additionné d'un seul acide aminé, comme cela est bien connu de l'homme de l'art.

La souche SA 103 selon l'invention que les inventeurs ont réussi à isoler est une souche extrêmement rare. En effet, une seule colonie a été mise en évidence sur plus de 120 échantillons analysés avec ou sans produits sélectifs tels que par exemple les antibiotiques cités précédemment.

Un autre objet de la présente invention est un procédé de production d'un bouillon de culture à partir d'une culture de la souche actinomycète *Saccharothrix* SA 103 et/ou d'au moins l'une de ses souches mutantes selon l'invention, **caractérisé en ce qu**'il comprend les étapes suivantes :
a) fermentation de ladite souche dans un bouillon nutritionnel afin d'obtenir le bouillon de culture ;
b) optionnellement, séparation du bouillon de culture obtenu à l'étape a).

La fermentation aérobie dans un milieu liquide est préférée, comme dans le cas de la production d'autres antibiotiques, et la production des composés actifs à partir de la souche *Saccharothrix* SA 103 selon l'invention peut être effectuée à n'importe quelle température favorable à la croissance de cette souche, c'est-à-dire allant de la température ambiante jusqu'à 43 °C. De préference, on utilise une température allant de 25 °C à 32 °C. De manière encore préférée, la température est de 30 °C. Cette culture peut durer plusieurs jours, par exemple de 2 à 10 jours. Normalement, le pH est légèrement alcalin, mais le pH exact peut varier selon le milieu de culture utilisé. On prélève la souche après croissance sur gélose, notamment inclinée, et optionnellement stockée à faible température, et on l'inocule dans un milieu liquide conventionnel constitué de substances nutritives semblables à celles décrites plus haut pour la croissance de la souche *Saccharothrix* SA 103 selon l'invention, sous agitation de manière à obtenir un bouillon de culture.

La fermentation peut être réalisée dans des flacons Erlenmeyer et dans des fermentateurs industriels ou de laboratoire ayant diverses capacités. Lorsque l'on procède à la fermentation dans une cuve, il est souhaitable de produire un inoculum dans un bouillon nutritif en inoculant le bouillon nutritif avec un prélèvement d'une culture inclinée ou à plat, ou une culture lyophilisée de l'organisme. Après avoir obtenu de cette manière un inoculum, celui-ci est transféré de manière aseptisée dans le milieu de la cuve de fermentation pour une production à grande échelle des composés actifs. Le milieu dans lequel l'inoculum est produit peut être le même ou peut être différent de celui utilisé dans la cuve, pour autant qu'une croissance correcte du microorganisme soit obtenue.

Le bouillon nutritionnel selon l'invention contient de manière générale le même type de substances nutritives que celles du milieu de culture de la souche *Saccharothrix* SA 103 selon l'invention tel que décrit plus haut (source de carbone, source d'azote...). Il peut également contenir des agents anti-mousse tels que de la paraffine liquide, de l'huile de soja, des graisses ou du silicone.

L'étape b) optionnelle de séparation du bouillon de culture peut être effectuée par n'importe quel procédé bien connu de l'homme de l'art, seul ou associé à un autre procédé de séparation, tel que par exemple une centrifugation, une filtration ou une pasteurisation.

Ainsi, de préférence, le procédé de production d'un bouillon de culture selon l'invention est **caractérisé en ce que** la sépararation réalisée à l'étape b) optionnelle est une centrifugation et/ou une filtration et/ou une pasteurisation.

L'invention a encore pour objet un bouillon de culture susceptible d'être obtenu selon le procédé selon l'invention.

A partir du bouillon de culture obtenu selon le procédé de production de l'invention, on procède à l'extraction des composés actifs. Pour cela, on utilise un solvant organique, tel que par exemple, mais sans s'y limiter, le n-butanol, l'acétate d'éthyle, le dichlorométhane, le n-propanol ou le 2-propanol. Après l'étape d'extraction, la déshydratation de la phase organique peut être réalisée de manière facultative afin d'éliminer de nombreuses impuretés polaires dans le concentrat actif, ce qui facilitera ultérieurement la purification finale du concentrat actif par HPLC. Pour cela, on peut utiliser du sulfate de sodium anhydre ou du sulfate de magnésium, et/ou on procède au séchage *in vacuo* de la phase organique. L'utilisation d'une filtration sur gel (gel dextran réticulé), d'une chromatographie sur colonne cellulose, d'une résine échangeuse d'ions ou d'une chromatographie sur couche mince (gel de silice) peut également être envisagée.

De telles méthodes sont bien connues de l'homme de l'art, qui saura utiliser ces différentes techniques seules ou combinées, de la manière la plus appropriée en vue de récupérer un concentrat actif à partir du bouillon de culture de la souche *Saccharothrix* SA 103 selon l'invention.

Ainsi, encore un autre objet de la présente invention est un procédé de production d'un concentrat actif à partir du bouillon de culture selon l'invention, **caractérisé en ce qu**'il comprend les étapes suivantes :
a) extraction organique du bouillon de culture avec un solvant organique ;
b) optionnellement, déshydratation de la phase organique obtenue et/ou séchage *in vacuo* ;
c) optionnellement, mise en suspension du concentrat actif, de préférence filtration de la suspension obtenue, et répétition des étapes a) et b) d'extraction organique et de déshydratation.

Le concentrat actif susceptible d'être obtenu selon le procédé de la présente invention constitue encore un objet de la présente invention.

Le concentrat actif ainsi obtenu est ensuite identifié au moyen d'analyses instrumentales telles que le spectre d'absorption des rayons visibles/ultraviolets, le spectre d'absorption des rayons infrarouges, le spectre RMN-¹H et le spectre RMN-¹³C, la spectrométrie de masse, et également l'analyse chromatographique (colonnes chromatographiques gel de silice ou gel dextran, résines échangeuses d'ions, chromatographie en phase liquide, chromatographie liquide haute performance en phase inverse ou HPLC, etc...). Ceci permet de caractériser et de produire les différents composés actifs, également dénommés « fractions actives », susceptibles d'être contenus dans le concentrat actif obtenu.

Ainsi, l'invention a encore pour objet un procédé de production d'un composé actif à partir du concentrat actif selon l'invention par chromatographie liquide haute performance en phase inverse (reverse phase HPLC), de préférence précédée d'une chromatographie sur couche mince et/ou d'une chromatographie liquide à basse pression.

Les composés actifs qui sont susceptibles d'être obtenus par le procédé de production selon l'invention correspondent donc à des éluats obtenus par chromatographie HPLC et sont quasiment purs.

La purification par chromatographie HPLC est indispensable pour séparer les composés actifs à partir du concentrat actif. La chromatographie sur couche mince et/ou la chromatographie liquide à basse pression telle que du type Sephadex LH 20 permet l'obtention d'un concentrat actif débarrassé de nombreuses impuretés et facilite ainsi la purification ultérieure par chromatographie HPLC.

De préférence, le procédé de production d'un composé actif selon l'invention est **caractérisé en ce que** le composé actif est une mutactimycine telle que la mutactimycine P11, la mutactimycine PR, la mutactimycine F ou la mutactimycine G, ou une aldgamycine telle que l'aldgamycine G, l'aldgamycine H ou l'aldgamycine P10b ou les sels d'addition pharmaceutiquement acceptables, isomères, énantiomères, diastéréoismères, ainsi que les mélanges de ces composés actifs.

La mutactimycine P11 possède la formule suivante :

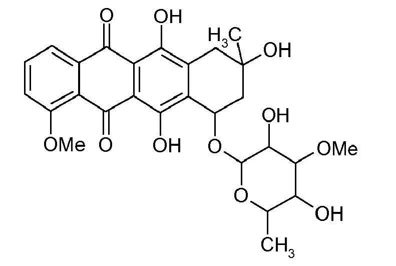

Elle a été identifiée comme correspondant à la mutactimycine C, déjà connue et enregistrée dans la base « Registry » sous le numéro RN 138689-81-3.

La mutactimycine PR possède la formule suivante :

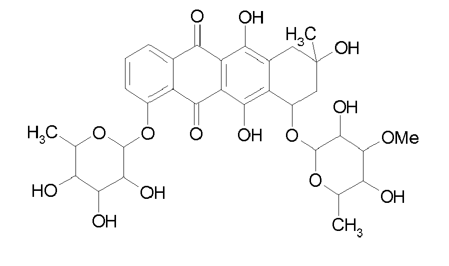

La mutactimycine F possède la formule suivante :

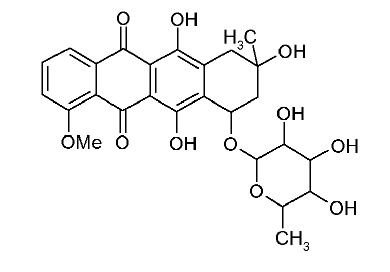

La mutactimycine G possède la formule suivante :

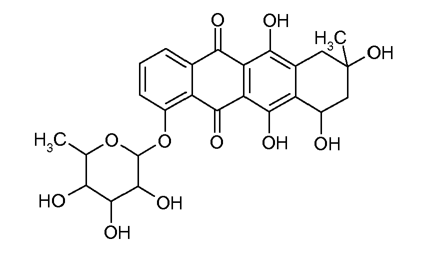

L'aldgamycine G (ou P10a) possède la formule suivante :

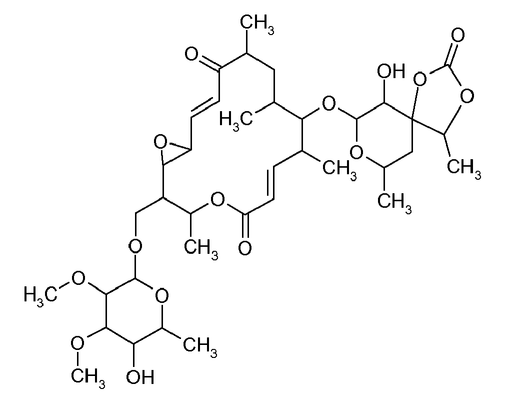

On connaît déjà l'aldgamycine de formule stéréochimique suivante, enregistrée dans la base « Registry » sous le numéro RN 107745-56-2.

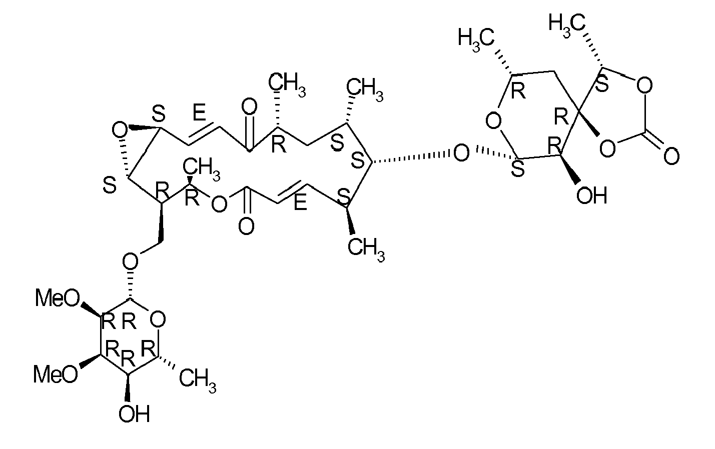

L'aldgamycine H (ou P8), possède la formule suivante :

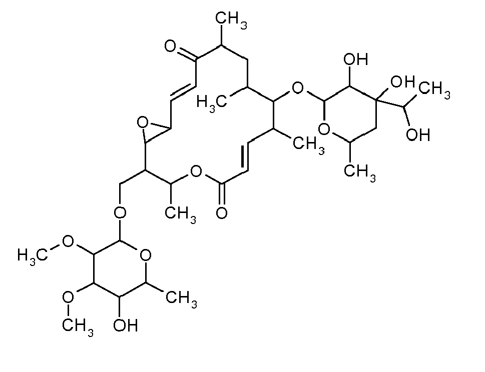

L'aldgamycine P10b (ou swalpamycine B) possède la formule suivante :

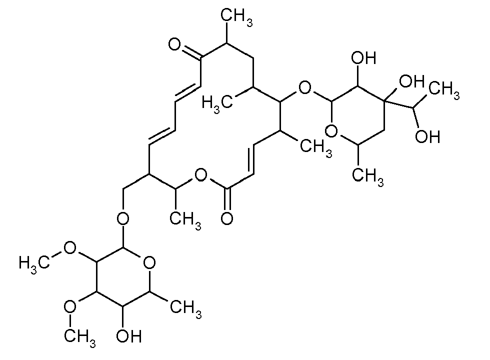

L'invention a également pour objet un composé actif susceptible d'être obtenu par le procédé de production selon l'invention, **caractérisé en ce que** le composé actif est la mutactimycine PR de formule suivante : 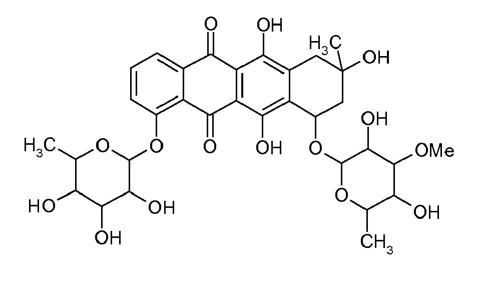 ou ses sels d'addition pharmaceutiquement acceptables, isomères, énantiomères, diastéréoismères, ainsi que leurs mélanges.

L'invention a également pour objet un composé actif susceptible d'être obtenu par le procédé de production selon l'invention, **caractérisé en ce que** le composé actif est la mutactimycine F de formule suivante : 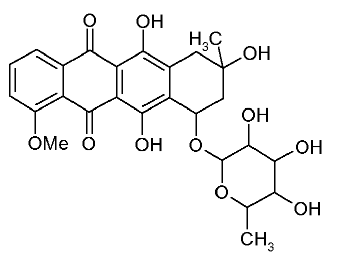 ou ses sels d'addition pharmaceutiquement acceptables, isomères, énantiomères, diastéréoismères, ainsi que leurs mélanges.

L'invention a également pour objet un composé actif susceptible d'être obtenu par le procédé de production selon l'invention, **caractérisé en ce que** le composé actif est la mutactimycine G de formule suivante : 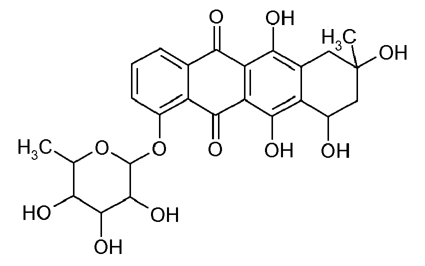 ou ses sels d'addition pharmaceutiquement acceptables, isomères, énantiomères, diastéréoismères, ainsi que leurs mélanges.

L'invention a également pour objet un composé actif susceptible d'être obtenu par le procédé de production selon l'invention, **caractérisé en ce que** le composé actif est l'aldgamycine G de formule suivante : 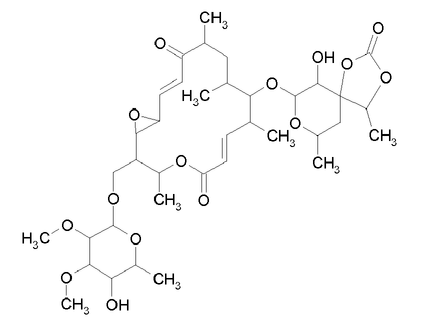 ou ses sels d'addition pharmaceutiquement acceptables, isomères, énantiomères, diastéréoismères, ainsi que leurs mélanges,
à l'exception de l'aldgamycine G de formule stéréochimique suivante :

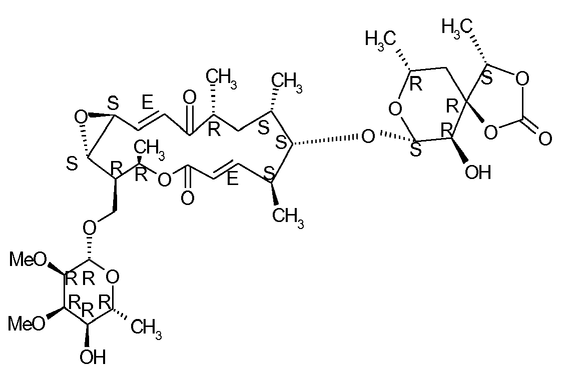

L'invention a également pour objet un composé actif susceptible d'être obtenu par le procédé de production selon l'invention, **caractérisé en ce que** l'aldgamycine G possède la formule stéréochimique suivante : 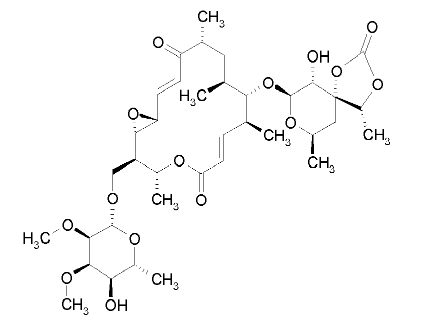 ou ses sels d'addition pharmaceutiquement acceptables, isomères, énantiomères, diastéréoismères, ainsi que leurs mélanges.

L'invention a également pour objet un composé actif susceptible d'être obtenu par le procédé de production selon l'invention, **caractérisé en ce que** le composé actif est l'aldgamycine H de formule suivante : 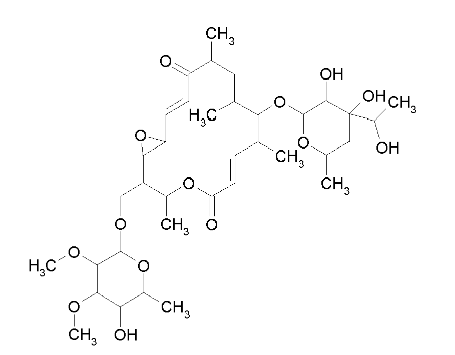 ou ses sels d'addition pharmaceutiquement acceptables, isomères, énantiomères, diastéréoismères, ainsi que leurs mélanges.

De préférence, l'aldgamycine H possède la formule stéréochimique suivante : 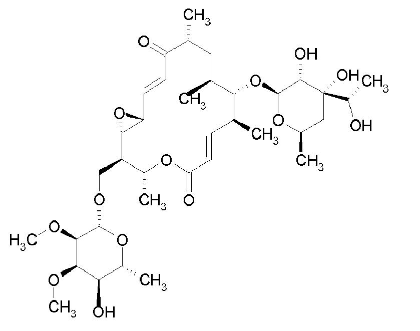 ou ses sels d'addition pharmaceutiquement acceptables, isomères, énantiomères, diastéréoismères, ainsi que leurs mélanges.

L'invention a également pour objet un composé actif susceptible d'être obtenu par le procédé de production selon l'invention, **caractérisé en ce que** le composé actif est l'aldgamycine P10b de formule suivante : 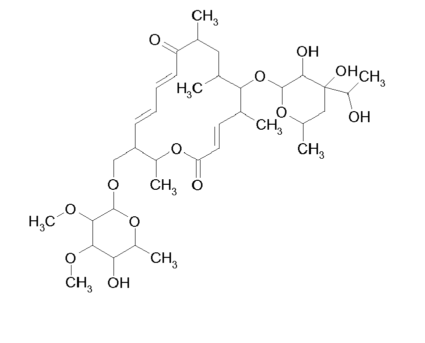 ou ses sels d'addition pharmaceutiquement acceptables, isomères, énantiomères, diastéréoismères, ainsi que leurs mélanges.

De préférence, l'aldgamycine P10b possède la formule stéréochimique suivante : 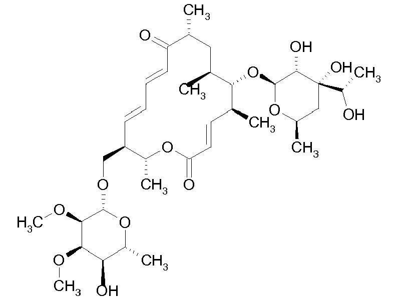 ou ses sels d'addition pharmaceutiquement acceptables, isomères, énantiomères, diastéréoismères, ainsi que leurs mélanges.

La mutactimycine PR, la mutactimycine F, la mutactimycine G, l'aldgamycine G, l'aldgamycine H ou l'aldgamycine P10b selon l'invention peut être obtenue par n'importe quel procédé connu de l'homme de l'art, tel que par exemple des procédés chimiques ou enzymatiques. De préférence, on utilisera le procédé de production comprenant une étape de fermentation à partir de la *Saccharothrix* SA 103 selon l'invention tel que décrit plus haut.

Un autre objet de la présente invention est un mélange actif d'au moins deux composés actifs choisis parmi la mutactimycine P11, la mutactimycine PR, la mutactimycine F ou la mutactimycine G, ou une aldgamycine telle que l'aldgamycine G, l'aldgamycine H ou l'aldgamycine P10b.

Facultativement, le mélange actif selon l'invention peut contenir au moins deux composés actifs choisis parmi la mutactimycine P11, la mutactimycine PR, la mutactimycine F ou la mutactimycine G, ou une aldgamycine telle que l'aldgamycine G, l'aldgamycine H ou l'aldgamycine P10b, et un autre composé actif sur le plan antibactérien, antiviral, antiprolifératif ou anticancéreux connu de l'homme de l'art (antibiotiques, etc..).

Les composés actifs selon l'invention peuvent être utilisés seuls ou en combinaison avec d'autres agents microbiens, par exemple pour prévenir la croissance ou réduire le nombre des bactéries Gram-positives. Ainsi, ces composés sont utiles sur le plan médical, notamment dans un but antibactérien, antiviral ou anticancéreux.

Un autre objet de l'invention est une composition pharmaceutique contenant une quantité thérapeutiquement efficace d'un composé actif selon l'invention, tel que la mutactimycine PR, la mutactimycine F, la mutactimycine G ou l'aldgamycine G, l'aldgamycine H ou l'aldgamycine P10b, et un excipient pharmaceutiquement acceptable .

Les compositions pharmaceutiques peuvent être préparées sous n'importe quelle forme pharmaceutique appropriée pour le mode d'administration. Des exemples de telles compositions comprennent les compositions solides pour une administration orale telles que des comprimés, des capsules, des pilules, des poudres et des granules, des compositions liquides pour une administration orale telles que des solutions, des suspensions, des sirops et des préprarations pour une administration parentérale telles que des solutions stériles, des suspensions ou des émulsions.

Les composés actifs de la présente invention forment des sels d'addition, le cas échéant pharmaceutiquement acceptables avec des réactifs de formation de tels sels, bien connus de l'homme de l'art.

Les quantités préférées des composés actifs de la présente invention utilisées pourront varier en fonction du composé actif particulier utilisé, de la composition particulière formulée, du mode d'application et du site particulier, de l'hôte et de la maladie à traiter. En général, les composés actifs de l'invention sont injectés en intrapéritonéale, en intraveineuse, en sous-cutané ou local, ou administrés oralement. Divers facteurs qui modifient l'action du médicament seront pris en compte par l'homme du métier, par exemple l'âge, le poids corporel, le sexe, le régime, le temps d'administration, le taux d'excrétion, la condition du patient, les combinaisons de médicaments, les sensibilités à des réactions et la sévérité de la maladie. L'administration peut être réalisée de manière continue ou périodique dans la dose maximale tolérée.

Il est à noter que pour une utilisation en tant qu'agents antibactériens, les composés actifs sont en général administrés de manière à ce que la concentration de l'ingrédient actif soit supérieure à celle de la concentration minimale inhibitrice pour l'organisme particulier à traiter.

Ainsi, l'invention a pour objet un composé actif selon l'invention, tel que la mutactimycine PR, la mutactimycine F, la mutactimycine G ou l'aldgamycine G, l'aldgamycine H ou l'aldgamycine P10b pour son utilisation en tant que médicament.

L'invention a encore pour objet l'utilisation d'une composition pharmaceutique selon l'invention, pour la fabrication d'un antibiotique destiné à prévenir et/ou à traiter une infection impliquant une bactérie gram-positif, telle qu'une streptococcie, une infection néonatale, une infection urinaire, une endocardite, une pneumonie, une méningite, une otite, une listériose, la diphtérie, la tuberculose ou la lèpre.

L'invention a également pour objet une méthode de traitement et/ou de prévention d'une infection impliquant une bactérie gram-positif, telle qu'une streptococcie, une infection néonatale, une infection urinaire, une endocardite, une pneumonie, une méningite, une otite, une listériose, la diphtérie, la tuberculose ou la lèpre, au moyen d'une composition pharmaceutique selon l'invention.

Un autre objet est l'utilisation d'une composition pharmaceutique selon l'invention, pour la fabrication d'un médicament antiviral destiné à prévenir et/ou à traiter une infection impliquant le virus de l'immunodéficience acquise (SIDA), le virus de la vaccine, le coronavirus, le papillomavirus, le parvovirus, le virus de la fièvre catarrhale du mouton, le virus de la Dengue, le virus Ebola, ou encore la grippe, la variole, la rougeole, la rubéole, la varicelle, l'hépatite A, B, C, D ou E, la mononucléose, la fièvre jaune, l'encéphalite ou l'herpès.

L'invention a également pour objet une méthode de traitement et/ou de prévention d'une infection le virus de l'immunodéficience acquise (SIDA), le virus de la vaccine, le coronavirus, le papillomavirus, le parvovirus, le virus de la fièvre catarrhale du mouton, le virus de la Dengue, le virus Ebola, ou encore la grippe, la variole, la rougeole, la rubéole, la varicelle, l'hépatite A, B, C, D ou E, la mononucléose, la fièvre jaune, l'encéphalite ou l'herpès, au moyen d'une composition pharmaceutique selon l'invention.

L'invention a encore pour objet l'utilisation d'une composition pharmaceutique selon l'invention, pour la fabrication d'un médicament anticancéreux destiné à prévenir et/ou à traiter un sujet atteint d'un cancer, tel que le cancer du poumon, de l'utérus, du sein ou de l'ovaire, le cancer colorectal, la leucémie ou un sujet atteint d'une tumeur de la prostate, de la vessie, de la peau, du cerveau, de la gorge.

L'invention a également pour objet une méthode de traitement et/ou de prévention d'un sujet atteint d'un cancer, tel que le cancer du poumon, de l'utérus, du sein ou de l'ovaire, le cancer colorectal, la leucémie ou un sujet atteint d'une tumeur de la prostate, de la vessie, de la peau, du cerveau, de la gorge, au moyen d'une composition pharmaceutique selon l'invention.

L'invention a également pour objet une méthode de prévention et/ou de traitement d'une maladie chez une plante au moyen d'un produit phytopharmaceutique comprenant un composé actif selon l'invention, tel que la mutactimycine PR, la mutactimycine F, la mutactimycine G ou l'aldgamycine G, l'aldgamycine H ou l'aldgamycine P10b.

Les composés actifs selon l'invention tels que la mutactimycine PR, la mutactimycine F, la mutactimycine G ou l'aldgamycine G, l'aldgamycine H ou l'aldgamycine P10b peuvent encore être utilisés pour traiter dans l'industrie alimentaire, du cuir ou du bois.

Ils sont également utiles dans des solutions de lavage dans un but sanitaire, par exemple pour le lavage des mains et la désinfection de divers équipements de laboratoire, dentaires et médicaux ou d'autres matériaux contaminés.

Les légendes des figures et exemples qui suivent sont destinées à illustrer l'invention sans aucunement en limiter la portée.

### LEGENDES DES FIGURES

Figure 1 : Profil de fermentation de la souche *Saccharothrix* sp. SA 103.
Figure 2 : Isolement et purification des produits actifs de la souche *Saccharothrix* sp. SA 103.

### EXEMPLES

### Exemple 1 : Matériels et méthodes

### 1.1 Micro-organisme

La souche productrice actinomycète *Saccharothrix* SA 103 selon l'invention a été isolée à partir d'un sol des régions arides de l'Algérie (Sahara) par mise en suspension d'un échantillon de sol dans de l'eau distillée stérile et dépôt sur une gélose humique-vitamines B contenant 50 µg/ml d'actidione. Une culture pure de la souche SA 103 a été préservée par lyophilisation. Elle a également été maintenue à 6°C à des fins d'utilisation au sein du laboratoire sur un milieu ISP N°2 incliné (Shirling et Gottlieb, Int. J. Syst. Bacteriol. 16 :313-340, 1996).

### 1.2 Taxonomie

Les caractéristiques taxonomiques de la souche SA 103 ont été déterminées par culture sur divers milieux décrits dans Shirling et Gottlieb (1996) et dans Waksman (« The actinomycetes », Vol. II, The Williams & Wilkins Co., Baltimore, 1961). Les caractéristiques morphologiques ont été déterminées après croissance à 30°C pendant 14 jours. Les noms de couleur et numéros de nuance ont été attribués en utilisant le système ISCC-NBS (« Inter Society Color Council » à l'intention du « National Bureau of Standards »). L'observation détaillée des morphologies du mycélium et des spores a été réalisée par microscopie électronique par balayage (Hitachi, modèle S-450). Les propriétés physiologiques ont été examinées à l'aide des méthodes Goodfellow (J. Gen. Microbiol. 69 :33-90, 1971) et Waksman (1961). Le type d'isomères de l'acide diaminopimélique dans la paroi cellulaire, et la composition cellulaire totale en sucres ont été déterminées par les méthodes de Becker et al. (Appl. Microbiol. 12 :421-423, 1964), et Lechevalier et Lechevalier (« In The Actinomycetales », Ed. H. Prauser, pp. 311-316, Fisher Verlag, Jena, 1970). Les phospholipides et les acides mycoliques ont été analysés par la procédure de Minnikin et al. (Int. J. Syst. Bacteriol. 27 :104-107, 1977 ; J. Chromatography 188 :221-233, 1980).

### 1.3 Fermentation

Un prélèvement de la souche SA 103 à partir d'une culture inclinée mature a été inoculé dans un flacon Erlenmeyer contenant 50 ml de milieu de culture stérile contenant du glucose 0,4 %, un extrait de malt 1 % et un extrait de levure 0,4 % (ajusté au pH 7,2 avant stérilisation) et mis en culture sur un agitateur rotatif (250 rpm) à 30 °C pendant 2 jours. Pour la production d'antibiotiques, 3 ml du milieu de culture ont été transférés dans des flacons Erlenmeyer de 500 ml, chacun contenant 100 ml du milieu ci-dessus, et mis en culture pendant 10 jours en utilisant les mêmes conditions. La production de l'activité antibactérienne totale a été réalisée sur une gélose nutritive, par un test de diffusion sur gélose contre *Bacillus subtilis* ATCC 6633. L'inhibition de croissance a été examinée après 24 heures d'incubation à 30°C. L'activité antimicrobienne a été estimée en mesurant le diamètre de la zone d'inhibition. Le poids sec du mycélium a été déterminé dans des tubes Eppendorf remplis avec 1 ml de bouillon de culture homogénéisé et séché à 105°C pendant 24 heures (Pfefferle et al., J. Biotech. 80 :135-142, 2000).

### 1.4 Purification d'antibiotiques

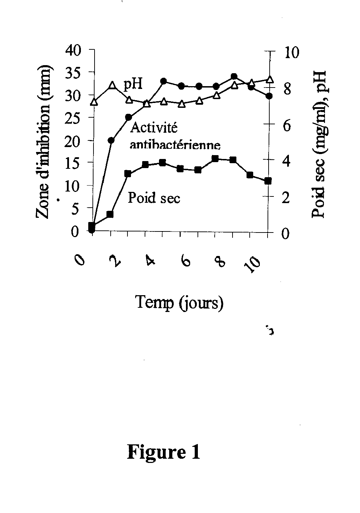

La production des antibiotiques antibactériens par la souche SA 103 est effectuée en milieu liquide MS + amidon (1 %) + extrait de levure (0,3 %) à pH 7,2 (100 ml de milieu par fiole Erlenmeyer de 500 ml, agitation à 250 rpm, incubation à 30 °C). Une cinétique a montré que la souche SA 103 commence à produire les antibiotiques dès le début de la phase exponentielle (1^{er} jour) et cette production se poursuit jusqu'au début de la phase stationnaire avant de se stabiliser pour décroître durant la phase de déclin (cf. Figure 1).

Au total, huit litres de culture ont été réalisées. Les filtrats sont extraits au n-butanol. L'extrait butanolique contenant les antibiotiques antibactériens recherchés est retenu.

L'extrait butanolique, de couleur rouge, est concentré au Rotavapor. Dans un premier temps, une aliquote de cet extrait a été passé à travers une colonne de Séphadex LH 20 en utilisant comme phase mobile du méthanol dans de l'eau bidistillée (80 %). Cependant, la séparation des antibiotiques (visualisation à l'oeil nu et par antibiographie) n'a pas été concluante. Cette étape a donc été supprimée et l'extrait butanolique a été purifié directement par HPLC en phase inverse en utilisant une colonne C18 et des conditions isocratiques (63 % de méthanol dans de l'eau), un débit de 1,5 ml/min et une détection à 220 nm. Les fractions correspondant à tous les pics obtenus sur le profil ont été recueillies séparément, concentrées et testées contre *Bacillus subtilis.* Sept fractions (7 pics) se sont révélées actives: quatre de couleur rouge vif (P11, PR, F et G) et trois non colorées (P8, P10a et P10b). Ces antibiotiques sont purifiés par HPLC après 3 à 4 réinjections dans les mêmes conditions que précédemment.

### Exemple 2 : Résultats et discussion - Caractérisation taxonomique de la souche productrice

### 2.1 Morphologie de la souche

La souche SA 103 a formé un mycélium aérien rose bien développé qui se fragmentait en chaînes de spores droites ou flexibles. Les spores étaient en forme de bâtonnets et avaient une taille de 1,9-2,9x0,6-0,7 µm avec une surface lisse (Figure 1). Les endospores, les granules sclérales, les synnemata et les spores à flagelles n'ont pas été observés. Le mycélium substrat était rouge brûnatre à rouge foncé et ne montrait pas ou peu de fragments. La souche a produit un pigment rouge foncé abondant caractéristique qui a été révélé comme correspondant à des antibiotiques antibactériens.

### 2.2 Caractéristiques structurales

Le tableau 1 montre les caractéristiques de culture de la souche SA 103 sur différents supports de culture. La croissance de la souche était abondante sur gélose d'extrait de levure et d'extrait de malt, gélose Bennett et gélose nutritive mais était modérée sur gélose flocons d'avoine et gélose sels inorganiques - amidon. La couleur du mycélium rentrait dans la gamme entre rose jaunâtre à marron rougeâtre clair pour les hyphes aériens et orange brûnatre à rouge très foncé pour le mycélium substrat. La souche produisait un pigment soluble rouge foncé ou orange brûnatre sur tous les milieux utilisés, mais aucun pigment mélanoïde n'a été observé.

L'espèce la moins différente est *Saccharothrix syringae* qui possède un mycélium aérien blanchâtre-rosâtre, un mycélium du substrat et des pigments solubles violet-rouge-brun.

### 2.3 Chimiotaxonomie

L'étude chimiotaxonomique a montré la présence d'acide meso-diaminopimélique et l'absence de glycine (paroi cellulaire de type III). Le motif en sucres de l'ensemble de la cellule a consisté en du rhamnose et du galactose (sucre cellulaire de type E) (Kroppenstedt, « The genus Nocardiopsis. In the Procaryotes » Ed., A. Balows et al., pp. 1139-1156, Springer Verlag, Berlin, 1192) et le phospholipide caractéristique était le phosphatidyl-éthanolamine (phospholipides de type PII). Aucun acide mycolique n'a été détecté.

### 2.4 Physiologie de la souche

Les résultats des tests physiologiques sont rapportés dans le tableau 2.

La souche est capable d'utiliser plusieurs composés organiques (caséine, gélatine, tween 80, amidon, tyrosine, etc.) dont la majorité des sucres. Par contre, à l'exception du mannitol, les dérivés alcooliques des oses (inositol, adonitol, dulcitol, érythritol et sorbitol) ne sont pas dégradés. La souche est capable de croître à 20 °C et 48 °C (avec un optimum à 30°C) et à pH 5 et 9 (avec un optimum entre 7 et 8). Elle est résistante au lysozyme et au violet cristal mais est sensible à tous les antibiotiques testés (11) à l'exception de la pénicilline et de la rifampicine.

La souche SA 103 se distingue de *Sa. syringae* par sa capacité à dégrader le lactose mais pas l'hypoxanthine et le butyrate de sodium, par sa sensibilité à l'érythromycine, la gentamicine, l'oxytétracycline et la vancomycine, par sa résistance à la pénicilline, la rifampicine et le violet cristal et par sa croissance à pH 5.

De ce fait, la souche SA 103 pourrait être une nouvelle espèce de *Saccharothrix* ou éventuellement une nouvelle sous-espèce de *Sa. syringae.*

### 2.5 Classification

En se basant sur les propriétés morphologiques et chimiques décrites plus haut, il a été considéré que la souche SA 103 appartenait au genre *Saccharothrix* (Labeda, et al, Int. J. Syst. Bacteriol. 34 :426-431, 1984). Comparée à l'espèce la plus proche *Saccharothrix syringae* NRRL B-16 468^{T}, la souche SA 103 différait par la capacité à dégrader le lactose mais pas l'hypoxanthine et le butyrate de sodium, par une susceptibilité à l'érythromycine, la gentamycine, l'oxytétracycline et la vancomycine, par une résistance à la pénicilline, la rifampicine, le crital violet et l'azoture de sodium, et par la croissance à pH 5,0. Ainsi, la souche a été désignée *Saccharothrix* sp. SA 103.

### 2.6 Fermentation

L'évolution au cours du temps de la production de l'activité antibactérienne par *Saccharothrix* sp. SA 103 est montrée dans la figure 1. La production de l'activité biologique contre *B. subtilis* a commencé lors du premier jour et était à son maximum au jour 4 pour devenir stable. La biomasse a augmenté lors des trois premiers jours, est restée stable, puis a diminué après le jour 8. La cinétique du pH a montré une augmentation remarquable lors du premier jour, puis est redevenue neutre, et a augmenté à la fin de la fermentation. De manière générale, la production des métabolites secondaires par les micro-organismes intervient pendant la phase stationnaire mais dans le présent cas la production de l'activité biologique était fortement corrélée à la croissance et était observée pendant toute l'évolution au cours du temps. La même cinétique de production au cours du temps a été observée pour la production d'antibiotiques dithiolopyrrolone par *Saccharothrix* sp. SA 233 (Lamari et al., J. Antibiotics 55 :696-701, 2002) et la production d'acide clavulanique par *Streptomyces clavuligerus* (Lebrihi et al., Appl. Microbiol. Biotechnol. 26 :130-135, 1987).

### 2.7 Détermination de la structure des antibiotiques

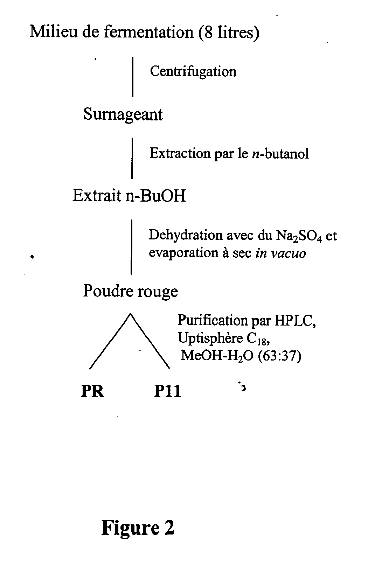

Les différentes étapes d'isolement et de purification d'antibiotiques sont résumés dans la figure 2.

### 2.7.1 Antibiotiques de la famille des anthracyclines,

Les antibiotiques P11, PR, F et G sont de couleur rouge vif à pH neutre, jaune à pH acide et violet-bleu à pH basique. P11 est l'antibiotique majoritaire et PR, l'antibiotique minoritaire. Ces antibiotiques ont fait l'objet d'études spectroscopiques suivantes: UV visible, infra-rouge, spectrométrie de masse et RMN du proton et du carbone 13 (avec études des corrélations). Des tests complémentaires, tels que la solubilité, la détermination des Rf par CCM et l'analyse élémentaire (cette dernière uniquement pour P11) ont été également effectués.

### Identification de la molécule P11 à la mutactimycine C

La molécule P11 possède un poids moléculaire de 530 et la formule chimique C₂₇H₃₀O₁₁. Les maxima obtenus dans l'UV-visible (MeOH) sont: 219, 234, 250, 287, 478, 496 et 531 nm. Le spectre est similaire à celui des antibiotiques de la famille des anthracyclines, de même que les changements de couleur à différents pH. Le spectre infra-rouge suggère la présence d'aromatiques, de groupements hydroxyles, méthyles et méthoxyles. Cependant, la structure finale a été élucidée après une étude détaillée par RMN du proton et du carbone 13 (déplacements chimiques, constantes de couplage spin-spin, intensités des signaux et corrélations ¹H-¹H cosy 45, ¹H-¹³C HMCQ et ¹H-¹³C HMBC). La molécule P11 a pu ainsi être identifiée à la mutactimycine C, connue pour être sécrétée par un mutant de *Streptomyces* sp. La mutactimycine C est constituée par le noyau anthracycline (4 cycles accolés dont un quinonique compris entre deux benzéniques), relié (par le biais du carbone C7) à un ose (le 6-deoxy-3-O-methyl-α-mannopyranoside). P11 est très soluble dans l'eau, le méthanol, le n-butanol, l'éthanol, le 1- propanol et l'acétone et insoluble dans le n-hexane et le toluène.

### Détermination de la structure chimique de nouvelles mutactimycines

### Mutactimycine PR

La molécule mineure PR possède un poids moléculaire de 662 qui correspond à la formule C₃₂H₃₈O₁₅. Les maxima obtenus dans l'UV-visible et les bandes dans l'infra-rouge sont identiques à ceux de P11 et les fragments de masse assez proches entre les deux antibiotiques, ce qui suggère une forte ressemblance dans la structure chimique. PR est très soluble dans l'eau, le méthanol et l'éthanol et insoluble dans le chloroforme, le dichlorométhane, l'acétate d'éthyle, le n-hexane et le toluène. La RMN du proton et celle du carbone 13 ont permis d'élucider la structure de PR qui s'est révélée être proche de P11 (mutactimycine C) avec comme seule différence la présence d'un second ose lié au carbone 4 du 1^{er} cycle benzénique (à la place de -OCH₃). Le second ose est le 6-deoxy-mannopyranoside (différence avec le premier par l'absence d'un méthyle). De par cette structure, la molécule PR appartient à la famille des anthracyclines et au groupe des mutactimycines. Cependant, elle diffère de toutes les mutactimycines connues et représente donc un nouvel antibiotique de ce groupe, nommé mutactimycine PR.

### Mutactimycine F

L'antibiotique F a un poids moléculaire de 516 (soit 14 de moins que P11) correspondant à une formule moléculaire C₂₆H₂₈O₁₁. Ses spectres UV-visible et infra-rouge et ses fragments de masse sont très proches de ceux de P11 et PR. Ceci suggère donc une forte ressemblance entre les trois molécules. Le composé F est soluble dans l'eau, le n-butanol, le 1-propanol et l'éthanol et insoluble dans le n-hexane et le toluène. Les RMN du proton et du carbone 13 ont permis d'établir la structure de la molécule qui s'est révélée être proche de P11 (mutactimycine C), mais avec comme seule différence la présence d'un OH sur le carbone C3 du résidu mannopyranosyl, au lieu d'un groupement -OCH3. L'antibiotique F est différent de toutes les mutactimycines connues et représente une nouvelle molécule dénommée mutactimycine F.

### Mutactimycine G

La molécule possède un poids moléculaire de 502 correspondant à une formule C₂₅H₂₆O₁₁. De fortes similitudes entre G, F, PR et P11 sont observées dans les spectres UV-visible et infra-rouge, ainsi que les fragments de masse. Le produit G est soluble dans l'eau, le n-butanol, le 1-propanol, le méthanol et l'éthanol et insoluble dans le chloroforme, le dichlorométhane, l'acétate d'éthyle, le n-hexane et le toluène. Les RMN du proton et du carbone 13 ont permis d'élucider la structure de cette molécule qui s'est révélée être proche de PR, avec comme seule différence l'absence du second ose lié au carbone C7 du 4^{éme} cycle du noyau anthracycline. L'antibiotique G représente donc une nouvelle molécule du groupe des mutactimycines et a ainsi été appelé mutactimycine G.

### 2.7.2 Antibiotiques de la famille des macrolides

Les antibiotiques P8, P10a et P10b ne sont pas colorés. P8 est la molécule majoritairement produite par *Saccharothrix* sp. SA 103 après P11 (mutactimycine C). Les trois antibiotiques sont très solubles dans le méthanol, le n-butanol, l'éthanol, le 1 et 2-propnol, l'acétate d'éthyle, l'acétone, le chloroforme et le dichlorométhane, mais insolubles dans l'eau et le n-hexane. Ces antibiotiques ont fait l'objet des mêmes études spectroscopiques que les mutactimycines décrites précédemment, ce qui a permis d'aboutir à la détermination de leurs structures chimiques.

### Identification de P10a à l'aldgamycine G

L'antibiotique P10a possède un poids moléculaire de 740 correspondant à la formule moléculaire C₃₇H₅₆O₁₅. Le spectre UV-visible et très semblable à celui des aldgamycines et a montré un maximum à 216 et deux épaulements à 245 et 278 nm. Le spectre infra-rouge présente plusieurs bandes d'absorptions qui indiquent la présence de groupements méthyle, méthoxyles, hydroxyles et une fonction carbonate. P10a est soluble dans le n-butanol, le 1 et 2-propanol, le méthanol, le dichlorométhane, l'acétate d'éthyle, le chloroforme, l'acétone et l'éthanol et insoluble dans le n-hexane, le toluène et l'eau. La molécule P10a a pu ainsi être identifiée à l'aldgamycine G, connue pour être sécrétée par *Streptomycs avidinii*. L'aldgamycine G est un macrolide neutre dont le cycle lactonique comporte 16 atomes sur lequel son reliés deux sucres méthylés, le mycinoe en position C14 et l'aldgarose en position C5.

### Détermination des structures chimiques des nouveaux macrolides

### Aldgamycine H

L'antibiotique P8 possède un poids moléculaire de 714, soit une formule moléculaire C₃₆H₅₈O₁₄. Ses propriétés physico-chimiques sont très semblable à celles de P10a (aldgamycine G). Cependant, le spectre UV-visible de P10b présente une intensité d'absorbance moindre à 245 et 278 nm et son spectre infra-rouge ne contient plus la bande d'absorption à 1800 cm⁻¹ caractéristique du groupement carbonate comme c'est le cas de l'aldgamycine G. Le produit P8, après analyse de toutes ses données spectroscopiques a été identifié à une nouvelle aldgamycine nommée H qui se rapproche de l'aldgamycine E, mais elle diffère par l'absence de la fonction carbonate sur le sucre aldgarose qui est hydrolysée dans ce cas.

### Swalpamycine B

La molécule P10b n'a pu être séparée du produit P10a par HPLC et de ce fait, elle a été analysé en même temps que le produit P10a sous forme de complexe. Elle possède un poids moléculaire de 698 et la formule chimique C₃₆H₅₈O₁₃. Les analyses RMN du proton et du carbone 13 ont permis d'élucider la structure finale. Ainsi P10a possède une structure chimique très semblable à celle de P8 et de la swalpamycine.

### 2.8 Activités microbiologiques des antibiotiques

L'activité microbiologique des mutactimycines P11, PR, F et G et des macrolides P8, P10(a et b) est dirigée surtout contre les bactéries à Gram positif. Les bactéries les plus sensibles sont *Micrococcus luteus* et *Klebsiella pneumoniae* qui est la seule bactérie à Gram négatif à être sensible.

Les concentrations minimales inhibitrices (CMI) sont de 5 µg/ml pour *M. luteus* et *K. pneumoniae*, 10 µg/ml pour *Staphylococcus aureus* CIP 53156, 40 µg/ml pour *Bacillus subtilis*, 50 µg/ml pour *Listeria monocytogenes* et 75 µl/ml pour *Mycobacterium smegmatis.* Les nouvelles molécules PR, F et G sont actives contre les mêmes microorganismes (à l'exception de *L. monocytogenes*). L'ensemble des molécules n'a aucune action contre *S. aureus* CIP 7625, les bactéries à Gram négatif *Escherichia coli, Pseudomonas syringae* pathovar *syringae* et *Agrobacterium tumefasciens* et contre la levure *Saccharomyces cerevisiae* et le champignon filamenteux *Mucor romannianus.*

L'activité des macrolides (aldgamycines G et H) et swalpamycine B est plus importante que celles des mutactimycines. En effet, les CMI ne sont que de 0,1 µg/ml pour *K. pneumoniae*, 1 µg/ml pour *Bacillus subtilis, Micrococcus luteus* et *Staphylococcus aureus* CIP 53156 qui sont les plus sensibles et respectivement 30 et 50 µg/ml pour *Listeria monocytogenes* et *Mycobacterium smegmatis.* Les bactéries à Gram négatif (sauf *K. pneumoniae)* et les champignons sont résistants.

| **Test organisms** | **MIC (ug/ml) (swalpamycine)** |
|---|---|
| *Bacillus subtilis* ATCC 6633 | 1 |
| *Micrococcus luteus* ATCC 9314 | 1 |
| *Staphylococcus aureus* CIP 7625 | > 100 |
| *Staphylococcus aureus* CIP 53156 | 1 |
| *Listeria monocytogenes* CIP 82110 | 30 |
| *Mycobacterium smegmatis* ATCC 607 | 50 |
| *Klebsiella pneumoniae* CIP 82.91 | 0.1 |
| *Escherichia coli* ATCC 10536 | > 100 |
| *Pseudomonas syringae* No 1882 | > 100 |
| *Agrobacterium tumefasciens* No 2410 | > 100 |
| *Mucor ramannianus* NRRL 1829 | > 100 |
| *Saccharomyces cerevisiae* ATCC 4226 | > 100 |

| **Test organisms** | **MIC (µg/ml) of (aldgamycine H)** |
|---|---|
| *Bacillus subtilis* ATCC 6633 | 10 |
| *Micrococcus luteus* ATCC 9314 | 1 |
| *Staphylococcus aureus* CIP 7625 | > 100 |
| *Staphylococcus aureus* CIP 53156 | 5 |
| *Listeria monocytogenes* CIP 82110 | > 100 |
| *Mycobacterium smegmatis* ATCC 607 | 20 |
| *Klebsiella pneumoniae* CIP 82.91 | 0.5 |
| *Escherichia coli* ATCC 10536 | >100 |
| *Pseudomonas syringae* No 1882 | > 100 |
| *Agrobacterium tumefasciens* No 2410 | >100 |
| *Mucor ramannianus NRRL* 1829 | >100 |
| *Saccharomyces cerevisiae* ATCC 4226 | >100 |

**Tableau 1 : Caractéristiques culturales de Saccharothrix sp. SA 103 .**

| Milieu | Croissance | Mycélium aérien | Mycélium du substrat | Pigment diffusible |
|---|---|---|---|---|
| Extrait de levure- extrait de malt agar (ISP No 2) | Bonne | Abondant Modéré Rose jaunâtre (29) | Rouge très sombre (14) | Rouge noirâtre (16) |
| Farine d'avoine agar (ISP No 3) | Modérée | Modéré Rose jaunâtre pale (31) | Orange brunâtre (54) | Orange brunâtre (54) |
| Sels inorganique-amidon agar (ISP No 4) | Modérée | Modéré Rose brunâtre (33) | Orange brunâtre (54) | Orange brunâtre (54) |
| Bennett agar | Bonne | Abondant Brun rougeâtre clair (42) | Brun rougeâtre sombre (44) | Brun rougeâtre sombre (44) |
| Gélose nutritive | Bonne | Modéré à abondant Rose jaunâtre (29) | Rouge foncé (13) | Rouge foncé (13) |

**Tableau 2 : Caractéristiques physiologiques de la souche Saccharothrix sp. SA 103.**

| Dégradation de | | Réduction de nitrate | + |
|---|---|---|---|
| Adénine | - | Production de pigments | |
| Arbutine | + | mélanoides | - |
| Caséine | + | | |
| Gélatine | + | Décarboxylation du sodium : | + |
| Esculine | + | Acétate | - |
| Guanine | - | Benzoate | - |
| Hypoxanthine | - | Butyrate | + |
| Amidon | + | Citrate | - |
| Testostérone | + | Oxalate | + |
| Tween 80 | + | Propionate | + |
| Tyrosine | + | Pyruvate | + |
| Xanthine | - | Succinate | - |
| Adonitol | - | Tartrate | |
| L-Arabinose | + | Croissance à : | + |
| Cellobiose | + | 48°C | + |
| Dextrine | + | pH 5 | + |
| Dulcitol | - | pH 9 | |
| Erythritol | - | Tolérance au : | + |
| D-Fructose | + | Cristal violet (0.001%) | + |
| Galactose | + | Lysozyme (0.005%) | + |
| D-Glucose | + | Phénol (0.05%) | - |
| Glycérol | + | Phénol (0.1%) | + |
| Inositol | - | Tellurite de potassium (0.01%) | + |
| Lactose | - | Azide de sodium (0.001%) | - |
| Maltose | + | Azide de sodium (0.01%) | - |
| D-Mannitol | + | Chlorure sodium (5%) | |
| D-Mannose | + | Résistance au : | - |
| Mélézitose | - | Chloramphénicol (25 µg/ml) - | |
| Mélibiose | - | Cyclosérine (10 µg/ml) | - |
| α-Méthyle-D-glucoside | - | Erythromycine (10 µg/ml) - | |
| D-Raffinose | - | Gentamicine (10 µg/ml) - | |
| L-Rhamnose | + | Kanamycine (25 µg/ml) - | |
| Ribose | + | Novobiocine (10 µg/ml) | + |
| Sorbitol | - | Oxytétracycline (25 µg/ml) | + |
| Saccharose | + | Pénicilline (25 µg/ml) - | |
| Tréhalose | + | Rifampicine (5 µg/ml) - | |
| D-Xylose | + | Streptomycine (10 µg/ml) - | |
| | | Vancomycine (5 µg/ml) | |

**Tableau 3 : Spectre antimicrobien des antibiotiques PR et P11 :**

| Souches test | CMI (µg/ml) | |
|---|---|---|
| | PR | P11 |
| | **(1)** | **(2)** |
| *Bacillus subtilis* ATCC 6633 | 75 | 40 |
| *Micrococcus luteus* ATCC 9314 | 50 | 5 |
| *Staphylococcus aureus* CIP 7625 | >100 | >100 |
| *Staphylococcus aureus* CIP 53156 | 50 | 10 |
| *Listeria monocytogenes* CIP 82110 | >100 | 50 |
| *Mycobacterium smegmatis* ATCC 607 | >100 | 75 |
| *Klebsiella pneumoniae* CIP 82.91 | 40 | 5 |
| *Escherichia coli* ATCC 10536 | >100 | >100 |
| *Pseudomonas syringae* No 1882 | >100 | >100 |
| *Agrobacterium tumefaciens* No 2410 | >100 | >100 |
| *Mucor ramannianus* NRRL 1829 | >100 | >100 |
| *Saccharomyces cerevisiae* ATCC 4226 | >100 | >100 |

**Tableau 4. Propriétés physicochimiques des produits PR et P11 (mutactimycine).**

| | **mutactimycine PR** | **P11 (mutactimycine C)** |
|---|---|---|
| Apparence | Poudre rouge | Poudre rouge |
| Couleur dans H₂O | | |
| Acide | Jaune | Jaune |
| Neutre | Rouge | Rouge |
| Basique | Violet-bleu | Violet-bleu |
| Formule chimique | C₃₂H₃₈O₁₅ | C₂₇H₃₀O₁₁ |
| Poids moléculaire | 662 | 530 |
| Nano-ESI-MS (m/z) | | |
| Mode négatif | 660.8 [M-H]⁻, 514.9, 354, 337, 319.1, 291.1 | 528.8 [M-H]⁻, 351, 333, 315, 294.2, 293.1 |
| Mode positif | 685.2 [M+Na]⁺ 539, 507, 360.9, 342.9 | 553.1 [M+Na]⁺, 375, 356.9, 198.3 |
| UV λₘₐₓ nm dans MeOH | 219,234,250,287,478, 496, 531 | 219, 234, 250, 287, 478, 496, 531 |
| IR νₘₐₓ Cellule de diamant | 3393, 2969, 2932,2878 | 3393, 2969, 2932,2878 |
| (cm⁻¹) | 2841,2709,2360,2113, 1611,1583,1444,1408, 1379, 1352, 1285, 1238, 1213, 1133, 1110, 1070, 1048 | 2841,2709,2360,2113, 1611,1583,1444,1408, 1379, 1352, 1285, 1238, 1213, 1133, 1110, 1070, 1048 |
| Solubilité relative | | |
| Très soluble | MeOH, EtOH, H₂O | MeOH, Me₂CO, H₂O, *n*-BuOH, EtOH, *1*-PrOH |
| Moyennement soluble | Me₂CO, *n*-BuOH, *1*-PrOH, *2*-PrOH | CH₂Cl₂, CHCl₃, EtOAc, *2*-PrOH |
| Insoluble | CH₂Cl₂, CHCl₃ EtOAc, *n*-hexane, toluène | *n*-hexane, toluène |
| CCM (Valeur des Rf)^{a} | | |
| (I) | 0.16 | 0.44 |
| (II) | 0.58 | 0.64 |
| (III) | 0.82 | 0.80 |
| HPLC (Rt)^{b} | 31.74 min | 35.65 min |

| | | |
|---|---|---|
| ^{a}CCM sur gel de silice (Merck No 5715). (I): EtOAc-MeOH (100:15). (II): *n*-BuOH-CH₃COOH-H₂O (3:1:1). (III): MeOH-CH₂Cl₂ (4:1). ^{b}Conditions d'HPLC: Uptisphere C₁₈ UP5ODB (250x7.8 mm, d.i.), Phase mobile: isocratique à 63% MeOH in H₂O, Débit: 1.5 ml/min, Détection: UV (220 nm). | | |

**Tableau 5. Assignements des données RMN du ¹H et du ¹³C du produit PR dans du DMSO-d6 à 298 K et dans du DMF-d₇ à 278 K.**

| Position | DMSO | | DMF | |
|---|---|---|---|---|
| | δ_{H} | δ_{C} | δ_{H} | δ_{C} |
| 1 | 8.00 (d) | 121.0 | 7,96 (d) | 120.6 |
| 2 | 7.84 (br dd) | nd | 7.81 (dd) | 135.3 |
| 3 | 7.66 (br) | nd | 7.68 (d) | 124.1 |
| 4 | | nd | | 157.9 |
| 4a | | nd | | 122.6 |
| 7 | 4.89 (m) | 73.2 | 4.92 (m) | 73.7 |
| 8 | 2.10 (dd) / 1.92 (dd) | 43.3 | 2.16 (dd) / 1.98 (dd) | 43.6 |
| 9 | | 67.8 | | 67.7 |
| 10 | 2.83 (d) / 2.61 (d) | 38.3 | 2.84 (d) / 2.65 (d) | 38.2 |
| 12a | | nd | | 136.0 |
| 6-OH | nd | | nd | |
| 9-OH | 4.70 (br) | | nd | |
| 9-Me | 1.30 (s) | 29.7 | 1.32 (s) | 29.2 |
| 11-OH | nd | | nd | |
| 1' | 5.16 (br s) | 104.4 | 5.22 (br s) | 104.7 |
| 2' | 3.83 (m) | 67.0 | 3.92 (m) | 67.1 |
| 3' | 3.00 (dd) | 81.5 | 3.04 (dd) | 81.7 |
| 4' | 3.33 ^{a} | 71.0 | 3.44 (dd) | 72.0 |
| 5' | 3.64 (dq) | 70.2 | 3.70 ^{a} | 69.8 |
| 2'-OH | 4.88 | | nd | |
| 3'-OMe | 3.22(s) | 56.9 | 3.17 (s) | 56.6 |
| 4'-OH | 4.90 | | nd | |
| 5'-Me | 1.21 (d) | 18.7 | 1.17 (d) | 18.0 |
| 1" | 5.69 (br s) | 99.1 | 5.73 (br s) | 99.4 |
| 2" | 4.00 (m) | 71.0 | 4.09 (m) | 71.3 |
| 3" | 4.04 (dd) | 70.9 | 4.10 (dd) | 71.3 |
| 4" | 3.35 ^{a} | 72.4 | 3.42 (dd) | 72.2 |
| 5" | 3.52(dq) | 70.9 | 3.54 ^{a} | 70.8 |
| 2"-OH | 5.15 (br) | | nd | |
| 3"-OH | 4.89 (br) | | nd | |
| 4"-OH | 4.97 | | nd | |
| 5"-Me | 1.09 (d) | 18.7 | 1.05 (d) | 17.9 |

| | | | | |
|---|---|---|---|---|
| Seul les signaux détectés sont représentés. ^{a}Signal sous le HOD résiduel. nd: non détecté. | | | | |

**Tableau 6. Assignements des données RMN du ¹H et du ¹³C du produit P11 dans du DMSO-d6 à 298 K.**

| **Position** | **δ_{H}** | **δ_{C}** |
|---|---|---|
| 1 | 7.81 (m) | 119.4 |
| 2 | 7.83 (m) | 136.0 |
| 3 | 7.56 (m) | 119.1 |
| 4 | | 161.5 |
| 4a | | 120.8 |
| 5 | | 186.9 |
| 5a | | 111.1 |
| 6 | | 157.4 |
| 6a | | 136.0 |
| 7 | 4,86 (dd) | 73.5 |
| | 2.13 (dd) / 1.93 | |
| 8 | (dd) | 42.8 |
| 9 | | 68.2 |
| 10 | 2.76 (d) / 2.62 (d) | 37.4 |
| 10a | | 136.8 |
| 11 | | 155,4 |
| 11a | | 111.4 |
| 12 | | 187.4 |
| 12a | | 135.7 |
| 4-OMe | 3.95 (s) | 56.2 |
| 6-OH | 14.13 (s)^{a} | |
| 9-OH | 4.78 (s) | |
| 9-Me | 1.31 (s) | 28.1 |
| 11-OH | 13.19 (s)^{a} | |
| 1' | 5.12 (d) | 104.2 |
| 2' | 3.87 (m) | 67.1 |
| 3' | 3.02 (dd) | 81.2 |
| 4' | 3.32 (ddd) | 71.7 |
| 5' | 3.63 (dq) | 69.8 |
| 2'-OH | 4.82 (d) | |
| 3'-OMe | 3.23 (s) | 56.1 |
| 4'-OH | 4.91 (d) | |
| 5'-Me | 1.21 (d) | 17.0 |

| | | |
|---|---|---|
| ^{a}Signaux pouvant être interchangeables. | | |

**Tableau 7. Propriétés physico-chimiques du composé F.**

| | **Mutactimycine F** |
|---|---|
| apparence | Poudre rouge vif |
| Formule chimique | C₂₆H₂₈O₁₁ |
| Poids moléculaire | 516 |
| Nano-ESI-MS (m/z) | |
| Mode négatif | 514.9 [M-H]⁻ |
| | |
| Mode positif | 539.11 [M+Na]⁺ |
| | |
| UV λₘₐₓ nm ans MeOH | 219, 234, 252, 286, 473, 494, 531 |
| | |
| IR νₘₐₓ Cellule de diamant (cm⁻¹) | 3351, 2970, 2927,2854, 2360, 2336, |
| | 2114, 1981, 1798, 1610, 1582, 1444, |
| | 1406, 1381, 1356, 1271, 1240, 1215, |
| | 1137, 1091, 1069, 1048 |
| | |
| Solubilité relative | |
| Très soluble | *n*-BuOH, *1*-PrOH |
| | |
| Moyennement soluble | MeOH, EtOH |
| | |
| Insoluble | H₂O, CH₂Cl₂, CHCl₃ |
| | EtOAc, Me₂CO, *n*-hexane, toluene |
| | |
| CCM (valeur des Rf)^{a} | |
| (I) | 0.30 |
| (II) | 0.62 |
| (III) | 0.78 |
| HPLC (Rt)^{b} | 22.25 min |

| | |
|---|---|
| ^{a}CCM sur gel de silice (Merck No 5715). (I): EtOAc-MeOH (100:15). (II): *n-*BuOH- CH₃COOH-H₂O (3:1:1). (III): MeOH-CH₂Cl₂ (4:1). ^{b}Conditions d'HPLC: Uptisphere C₁₈ UP5ODB (250x7.8 mm, d.i.), Phase mobile: isocratique à 63% MeOH in H₂O, Débit: 1.5 ml/min, Détection: UV (220 nm). | |

**Tableau 8. Assignements des données RMN du ¹H et du ¹³C du produit F dans du DMSO-d6 à 298 K.**

| **Position** | **δ_{H}** | **δ_{C}** |
|---|---|---|
| 1 | 7.91 (m) | 120.5 |
| 2 | 7.90 (m) | 137.0 |
| 3 | 7.64 (m) | 119.8 |
| 4 | | 161.6 |
| 4a | | 119.8 |
| 5 | | 187.4 |
| 5a | | 111.4 |
| 6 | | 157.5 |
| 6a | | 137.3 |
| 7 | 4.90 (dd) | 73.2 |
| 8 | 2.13 (dd) / 1.94 (dd) | 43.3 |
| 9 | | 67.8 |
| 10 | 2.83 (d) / 2.64 (d) | 38.3 |
| 10a | | 137.0 |
| 11 | | 155.5 |
| 11a | | 111.5 |
| 12 | | 187.3 |
| 12a | | 135.7 |
| OMe-4 | 3.99 (s) | 57.5 |
| OH-6 | 14.16 (br s)^{a} | |
| OH-9 | 4.78 (s) | |
| Me-9 | 1.30 (s) | 29.8 |
| OH-11 | 13.26 (br s)^{a} | |
| 1' | 5.07 (d) | 104.6 |
| 2' | 3.61 (m) | 71.4 |
| 3' | 3.30 (ddd) | 71.5 |
| 4' | 3.24 (ddd) | 72.7 |
| 5' | 3.60 (dq) | 70.2 |
| OH-2' | 4.78 (d) | |
| OH-3' | 4.47 (d) | |
| OH-4' | 4.77 (d) | |
| Me-5' | 1.20 (d) | 19.8 |

| | | |
|---|---|---|
| ^{a}Signal sous le HOD résiduel. nd: non détecté. | | |

**Tableau 9. Propriétés physico-chimiques du composé G.**

| | **G** |
|---|---|
| Apparence | Poudre rouge vif |
| Formule chimique | C₂₅H₂₆O₁₁ |
| Poids moléculaire | 502 |
| Nano-ESI-MS (m/z) | |
| Mode négatif | 500.9 [M-H]⁻, 355, 337, 319.2, 291.2 |
| | |
| UV λₘₐₓ nm dans MeOH | 219, 234, 252, 286, 473, 494, 531 |
| | |
| IR νₘₐₓ Cellule de diamant | 3351, 2970, 2927,2854, 2360, 2336, |
| (cm⁻¹) | 2114, 1981, 1798, 1610, 1582, 1444, |
| | 1406, 1381, 1356, 1271, 1240, 1215, |
| | 1137, 1091, 1069, 1048 |
| | |
| Solubilité relative | |
| Très soluble | *n*-BuOH, *1*-PrOH, |
| | |
| Moyennement soluble | MeOH, EtOH, |
| | |
| Insoluble | H₂O, CH₂Cl₂, CHCl₃ |
| | EtOAc, Me₂CO, *n*-hexane, toluène |
| | |
| CCM (valeurs des Rf)^{a} | |
| (I) | 0.30 |
| (II) | 0.62 |
| (III) | 0.78 |
| | |
| HPLC (Rt)^{b} | 22.24 min |

| | |
|---|---|
| ^{a}CCM sur gel de silice (Merck No 5715). (I): EtOAc-MeOH (100:15). (II): *n*-BuOH- CH₃COOH-H₂O (3:1:1). (III): MeOH-CH₂Cl₂ (4:1). ^{b}Conditions d'HPLC: Uptisphere C₁₈ UP5ODB (250x7.8 mm, d.i.), Phase mobile: isocratique à 63% MeOH in H₂O, Débit: 1.5 ml/min, Détection: UV (220 nm). | |

**Tableau 10. Assignements des données RMN du ¹H et du ¹³C du produit G dans du DMSO-d6 à 298 K.**

| **Position** | **δ_{H}** | **δ_{C}** |
|---|---|---|
| 1 | 7.97 (dd) | 120.9 |
| 2 | 7.87 (dd) | 137.0 |
| 3 | 7.72 (dd) | 124.2 |
| 4 | | 157.8 |
| 4a | | 121.2 |
| 5 | | nd |
| 5a | | 111.1 |
| 6 | | nd |
| 6a | | 139,6 |
| 7 | 5.08 (dd) | 63.7 |
| | 2.12 (dd) / 1.74 | |
| 8 | (dd) | 44.3 |
| 9 | | 68.1 |
| 10 | 2.73 (br s) | 38.3 |
| 10a | | 136,6 |
| 11 | | 155.4 |
| 11a | | 111.4 |
| 12 | | 186.2 |
| 12a | | 135,5 |
| OH-6 | 14.20 (br s)^{a} | |
| OH-7 | 4.89 (s) | |
| OH-9 | 4.60 (s) | |
| Me-9 | 1.31 (s) | 30.7 |
| OH-11 | 13.30 (br s)^{a} | |
| 1' | 5.65 (br s) | 99.5 |
| 2' | 4.02 (m) | 70,6 |
| 3' | 4.02 (m) | 71.0 |
| 4' | 3.36 (m) | 72.5 |
| 5' | 3.55 (dq) | 70.9 |
| OH-2' | 5.15 (d) | |
| OH-3' | 4.86 (d) | |
| OH-4' | 4.96 (d) | |
| Me-5' | 1.10 (d) | 19.8 |

| | | |
|---|---|---|
| ^{a}Signaux pouvant être interchangeables. | | |

**Tableau 11: Assignements des données RMN du ¹H et du ¹³C du produit P8 (aldgamycine H) dans du CD₃OD et dans du DMSO-D₈ à 298K.**

| **Position** | **P8 (aldgamycin H)dans DMSO** | **P8 (aldgamycin H)dans CD3OD** | |
|---|---|---|---|
| | **dH (multi, J en Hz)** | **dH (multi, J en Hz)** | **dC** |
| 1 | | | 166.4 |
| 2 | 6.09 d (15.3) | 6.03 d (15.4) | 120.9 |
| 3 | 6.56 dd (15.3; 10.6) | 6.64 dd (15.4; 10.6) | 152.1 |
| 4 | 2.72 ddq (10.6; 10.2 ; 6.8) | 2.83 ddq (10.6; 10.2 ; 6.7) | 41.9 |
| 5 | 3.27 br d (10.2) | 3.38 brd (10.2) | 86.8 |
| 6 | 0.98 br m | 1.14 br m | 34.6 |
| | 1.40 ddd(14.4 , 12.0, 4.5) /1.77 | 1.59 ddd(14.0 , 12.0, 4.1) / 1.81 | |
| 7 | ddd(14.4, 11.0, 3.4) | ddd(14.0, 11.6, 3.1) | 32.0 |
| 8 | 2.41 ddq (12.0 ; 5.0; 6.9) | 2.56 ddq (11.9 ; 4.4; 7.0) | 45.2 |
| 9 | | | 202.8 |
| 10 | 7.02 d (15.4) | 6.92d(15.4) | 126.7 |
| 11 | 6.20 dd (15.4 ; 9.4) | 6.35 dd (15.4 ; 9.4) | 144.1 |
| 12 | 3.43 dd (9.4 ; 2.0) | 3.43 dd (9.4 ; 1.9) | 59.0 |
| 13 | 2.99 dd (9.3 ; 2.0) | 3.06 dd (9.3 ; 1.9) | 59.2 |
| 14 | 1.37 br m | 1.42 br m | 49.8 |
| 15 | 5.21 dq (10.8 ; 6.3) | 5.40 dq (10.9 ; 6.2) | 68.7 |
| 16 | 1.25 d (6.3) | 1.36 d (6.2) | 17.4 |
| 17 | 1.15 d (6.8) | 1.26 d (6.7) | 18.4 |
| 18 | 0.89 d (7.5) | 1.02 d (6.8) | 16.6 |
| 19 | 1.1 d (6.9) | 1.18 d (7.0) | 16.8 |
| | 3.59 dd (10.3 ; 2.8) / 3.94 dd | 3.70 dd (10.1 ; 2.7) / 4.14 dd | |
| 20 | (10.3,2.9) | (10.1 2.9) | 67.1 |
| 1' | 4.39 d (8.0) | 4.60 d (7.9) | 103.0 |
| 2' | 3.39 dd (8.0 ; 6.2) | 3.54 d (7.9) | 70.8 |
| 3' | | | 75.4 |
| | 1.32 dd (12.3 ; 8.3) /1.38 dd | 1.44 dd (11.8; 8.3) /1.55 dd (11.8 | |
| 4' | (12.3 ; 2.4) | ; 2.4) | 36.3 |
| 5' | 3.73 ddq (8.3 ; 2.4 ; 6.2) | 3.90 ddq (8.3 ; 2.4 ; 6.1) | 66.6 |
| 6' | 3.70 dq (6.3 ; 3.9) | 3.90 q (6.5) | 68.5 |
| OH-2' | 4.75 d (6.2) | nd | |
| OH-3' | 3.89 br s | nd | |
| Me-5' | 1.07 d (6.2) | 1.18 d (6.1) | 20.4 |
| OH-6' | 4.47 d (3.9) | nd | |
| Me-6' | 0.98 d (6.3) | 1.17 d (6.5) | 15.7 |
| 1" | 4.49 d (8.1) | 4.60 d (8.0) | 101.2 |
| 2" | 3.02 dd (8.1; 2.7) | 3.11 dd (8.0 ; 2.9) | 81.8 |
| 3" | 3.66 dd (2.7; 2.6) | 3.80 dd (2.9; 2.8) | 80.5 |
| 4" | 3.09 ddd (9.6 ; 7.1 ; 2.6) | 3.20 ddd (9.5; 2.8) | 73.6 |
| 5" | 3.54 dq (9.6 ; 6.2) | 3.69 dq (9.5 ; 6.3) | 70.1 |
| OMe-2" | 3.41 s | 3.57 s | 58.5 |
| OMe-3" | 3.46 s | 3.60 s | 61.1 |
| OH-4" | 4.88 d (7.1) | nd | |
| Me-5" | 1.12 d (6.2) | 1.25 d (6.3) | 17.1 |

| | | | |
|---|---|---|---|
| nd : non détecté | | | |

**Tableau 12. Propriétés physicochimiques des produits P8 et P10a.**

| | **P8** | **P10a** |
|---|---|---|
| Apparence | Poudre incolore | Poudre incolore |
| Formule chimique | C₃₆H₅₈O₁₄ | C₃₇H₅₆O₁₅ |
| | | |
| Poids moléculaire | 714 | 740 |
| Nano-ESI-MS (m/z) | | |
| Mode positif | 737.4 [M+Na]⁺, 563.3, | 763.44 [M+Na]⁺, |
| | 545.2, 389.2, 371.1, | 589.27, 563, 571.24, |
| | 289.1 | 559.19, 545.29, 417.16, |
| | | 389.16, 371.16, 289.02 |
| UV λₘₐₓ nm dans MeOH | 216.73, épaulements à | 216.73, épaulement |
| | 245.71 et 280 | 245.71 et 278.09 |
| | | |
| IR νₘₐₓ Cellule de diamant | 3431, 2970, 2931, 2880, | 3361, 2968, 2920, 2851, |
| (cm⁻¹) | 1712, 1689, 1654, 1620, | 1799, 1712, 1653, 1623, |
| | 1581, 1454, 1416, 1382, | 1593, 1562, 1508, 1457, |
| | 1354, 1326, 1279, 1263, | 1417,1383,1356,1324, |
| | 1236, 1172, 1159, | 1282, 1238, 1194, 1173, |
| | 1117,1082 | 1159, 1117, 1084, 1047 |
| | | |
| Solubilité relative | | |
| Très soluble | MeOH, EtOH, Me₂CO, | MeOH, EtOH, Me₂CO, |
| | *n*-BuOH, *1*-PrOH, 2- | *n*-BuOH, *1*-PrOH, 2- |
| | PrOH, CH₂Cl₂, EtOAc, | PrOH, CH₂Cl₂, EtOAc, |
| | CHCl₃ | CHCl₃ |
| | | |
| Insoluble | *n*-hexane, toluène, H₂O | *n*-hexane, toluène, H₂O |
| CCM (valeur du Rf)^{a} | | |
| (I) | 0.63 | 0.68 |
| (II) | 0.71 | 0.74 |
| (III) | 0.86 | 0.88 |
| | | |
| HPLC (Rt)^{b} | 23.21 min | 28.38 min |

| | | |
|---|---|---|
| ^{a}CCM sur gel de silice (Merck No 5715). (I): EtOAc-MeOH (100:15). (II): *n-*BuOH- CH₃COOH-H₂O (3:1:1). (III): MeOH-CH₂Cl₂ (4:1). ^{b}Conditions d'HPLC: Uptisphère C₁₈ UP5ODB (250x7.8 mm, d.i.), Phase mobile: isocratique à 63% MeOH in H₂O, Débit: 1.5 ml/min, Détection: UV (220 nm). | | |

**Tableau 13. Assignements des données RMN du ¹H et du ¹³C du produit P10a (aldgamycin G) dans du CD₃OD à 298K.**

| **Position** | **P10a (aldgamycin G)** | |
|---|---|---|
| | **δH (multi, J dans Hz)** | **δC** |
| 1 | | 166.3 |
| 2 | 6.03 d (15.5) | 121.0 |
| 3 | 6.64dd(15.5;10.7) | 151.8 |
| 4 | 2.84 ddq (10.7; 10.2; 6.8) | 41.8 |
| 5 | 3.42 d (10.2) | 86.8 |
| 6 | 1.16 m | 34.5 |
| 7 | 1.56 m / 1.82 ddd(14.0, 11.8,2.9) | 31.9 |
| 8 | 2.54 ddq (11.8 ; 4.8; 7.0) | 45.2 |
| 9 | | 202.7 |
| 10 | 6.92 d (15.4) | 126.6 |
| 11 | 6.35 dd (15.4 ; 9.4) | 144.2 |
| 12 | 3.43 dd (9.4 ; 2.1) | 59.0 |
| 13 | 3.06 dd (9.3 ; 2.1) | 59.2 |
| 14 | 1.43 dm (10.8) | 49.8 |
| 15 | 5.40 dq (10.8 ; 6.3) | 68.7 |
| 16 | 1.36 d (6.3) | 17.4 |
| 17 | 1.24 d (6.8) | 18.2 |
| 18 | 1.01 d (6.8) | 16.6 |
| 19 | 1.18 d (7.0) | 16.8 |
| 20 | 3.70 dd(10.2 ; 3.1) / 4.13 dd(10.2, 2.9) | 67.1 |
| 1' | 4.59 d (7.7) | 102.4 |
| 2' | 3.47 d (7.7) | 70.6 |
| 3' | | 86.6 |
| 4' | 1.61 dd(14.6; 11.1)/1.92dd(14.6;2.1) | 41.1 |
| 5' | 3.84ddq(11.1;6.1;2.1) | 67.0 |
| 6' | 4.50 q (6.5) | 82.2 |
| 7' | | 155.6 |
| OH-2' | nd | |
| Me-5' | 1.23d(6.1) | 19.9 |
| Me-6' | 1.57 d (6.5) | 12.6 |
| 1" | 4.60 d (8.0) | 101.2 |
| 2" | 3.11 dd (8.0 ; 2.8) | 81.8 |
| 3" | 3.80 dd (2.8 ; 2.7) | 80.5 |
| 4" | 3.20 dd (9.6 ; 2.7) | 73.6 |
| 5" | 3.69 dq (9.6 ; 6.2) | 70.1 |
| OMe-2" | 3.57 s | 58.5 |
| OMe-3" | 3.60 s | 61.1 |
| OH-4" | nd | |
| Me-5" | 1.25 d (6.2) | 17.1 |

| | | |
|---|---|---|
| nd : non détecté | | |

**Tableau 14. Assignements des données RMN du ¹H et du ¹³C du produit P10b (swalpamycine B) dans du CD₃OD à 298K.**

| **Position** | **P10b (swalpamycine B)** | |
|---|---|---|
| | **δH (multi, J dans Hz)** | **δC** |
| 1 | | 166.7 |
| 2 | 5.88 d (15.4) | 121.4 |
| 3 | 6.62 dd (15.4 ; 10.0) | 152.5 |
| 4 | 2.79 m | 41.3 |
| 5 | 3.36 br d (10.1) | 87.3 |
| 6 | 1.21 m | 34.5 |
| 7 | 1.59 m^{a} / 1.70 ddd(14.4, 11.7, 3.2) | 31.9 |
| 8 | 2.53 m | 45.3 |
| 9 | | 205.7 |
| 10 | 6.46 d (15.0) | 123.9 |
| 11 | 7.08 dd (15.0 ; 10.9) | 142.4 |
| 12 | 6.28 dd (15.2 ; 10.9) | 133.4 |
| 13 | 6.08 dd (15.2 ; 9.3) | 142.0 |
| 14 | 2.46 m | 51.5 |
| 15 | 5.12 dq (10.1 ; 6.3) | 69.7 |
| 16 | 1.39 d (6.8) | 17.6 |
| 17 | 1.22 d (6.3) | 19.1 |
| 18 | 1.01 d (6.8) | 16.9 |
| 19 | 1.18 d (7.0) | 17.0 |
| 20 | 3.66 dd(9.9 ; 3.6) / 4.01 dd(9.9 , 3.4) | 68.5 |
| 1' | 4.56 d (8.0) | 102.9 |
| 2' | 3.54 d (8.0) | 70.8 |
| 3' | | 75.4 |
| 4' | 1.46 m / 1.54 m | 36.3 |
| 5' | 3.87 ddq (8.3 ; 6.2 ; 2.4) | 66.6 |
| 6' | 3.90 q (6.6) | 68.5 |
| OH-2' | nd | |
| OH-3' | nd | |
| Me-5' | 1.19 d (6.2) | 20.4 |
| OH-6' | nd | |
| Me-6' | 1.16 d (6.6) | 15.6 |
| 1" | 4.61 d (8.0) | 101.3 |
| 2" | 3.08 dd (8.0 ; 2.9) | 81.8 |
| 3" | 3.77 dd (2.9 ; 2.8) | 80.6 |
| 4" | 3.19 dd (9.6 ; 2.9) | 73.6 |
| 5" | 3.67 dq (9.6 ; 6.3) | 70.0 |
| OMe-2" | 3.53 s | 58.6 |
| OMe-3" | 3.59 s | 61.1 |
| OH-4" | nd | |
| Me-5" | 1.24 d (6.3) | 17.1 |

| | | |
|---|---|---|
| nd : non détecté | | |

## Revendications

1. Mutactimycine G de formule suivante : 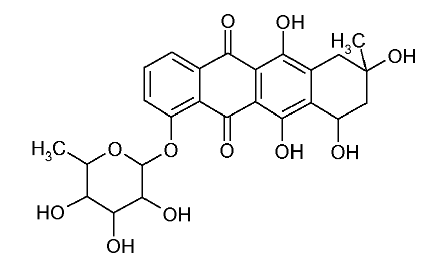 ou ses sels d'addition pharmaceutiquement acceptables, isomères, énantiomères, diastéréoismères, ainsi que leurs mélanges.

2. Mutactimycine F de formule suivante : 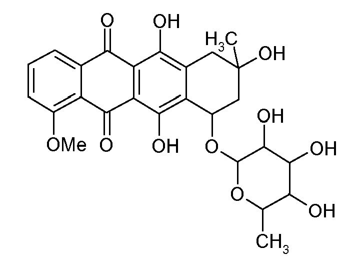 ou ses sels d'addition pharmaceutiquement acceptables, isomères, énantiomères, diastéréoismères, ainsi que leurs mélanges.

3. Mutactimycine PR de formule suivante : 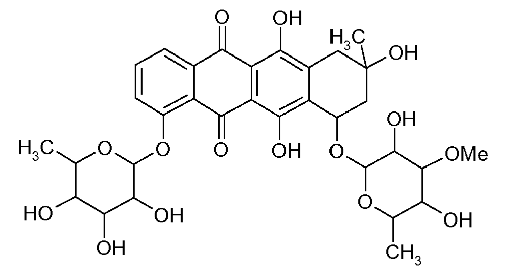 ou ses sels d'addition pharmaceutiquement acceptables, isomères, énantiomères, diastéréoismères, ainsi que leurs mélanges.

4. Aldgamycine G de formule stéréochimique suivante : 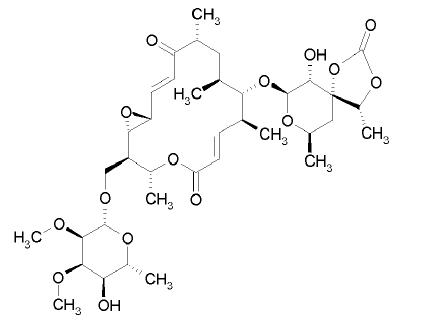 ou ses sels d'addition pharmaceutiquement acceptables, isomères, énantiomères, diastéréoismères, ainsi que leurs mélanges.

5. Aldgamycine H de formule suivante : 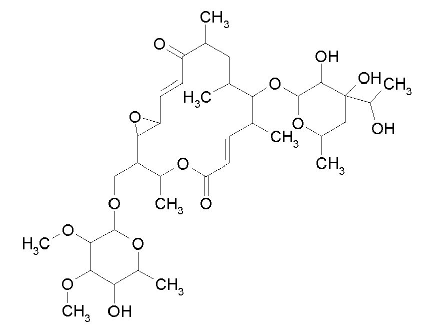 ou ses sels d'addition pharmaceutiquement acceptables, isomères, énantiomères, diastéréoismères, ainsi que leurs mélanges.

6. Aldgamycine H selon la revendication 5, **caractérisé en ce qu'**elle possède la formule stéréochimique suivante : 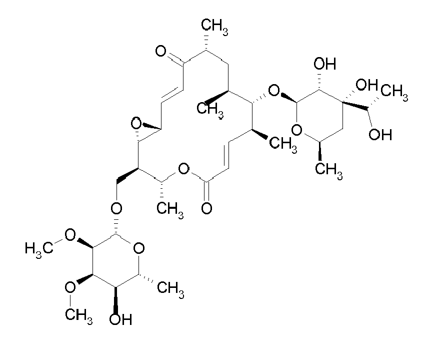 ou ses sels d'addition pharmaceutiquement acceptables, isomères, énantiomères, diastéréoismères, ainsi que leurs mélanges.

7. Aldgamycine P10b, de formule suivante : 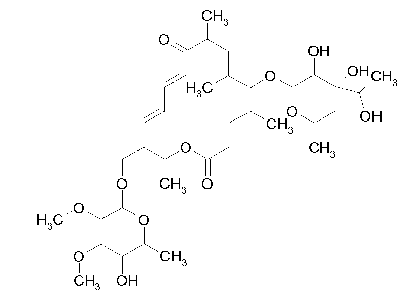 ou ses sels d'addition pharmaceutiquement acceptables, isomères, énantiomères, diastéréoismères, ainsi que leurs mélanges.

8. Aldgamycine P10b selon la revendication 7, **caractérisé en ce qu'**elle possède la formule stéréochimique suivante : 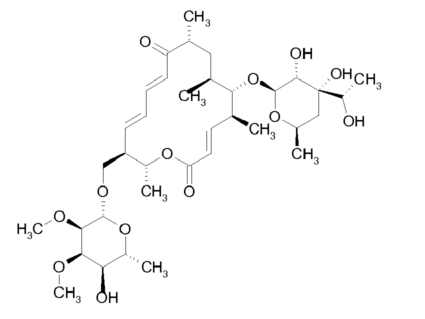 ou ses sels d'addition pharmaceutiquement acceptables, isomères, énantiomères, diastéréoismères, ainsi que leurs mélanges.

9. Composition pharmaceutique contenant une quantité thérapeutiquement efficace de Mutactimycine PR, Mutactimycine F, Mutactimycine G ou d'Adgamycine G, d'Aldgamycine H ou d'Aldgamycine P10b telles que définies aux revendications 1 à 8 et un excipient pharmaceutiquement acceptable.

10. Mutactimycine ou Aldgamycine telle que définie aux revendications 1 à 8, pour son utilisation en tant que médicament.

11. Utilisation d'une composition pharmaceutique selon la revendication 9, pour la fabrication d'un antibiotique destiné à prévenir et/ou à traiter une infection impliquant une bactérie gram-positif, telle qu'une streptococcie, une infection néonatale, une infection urinaire, une endocardite, une pneumonie, une méningite, une otite, une listériose, la diphtérie, la tuberculose ou la lèpre.

12. Utilisation d'une composition pharmaceutique selon la revendication 9, pour la fabrication d'un médicament antiviral destiné à prévenir et/ou à traiter une infection impliquant le virus de l'immunodéficience acquise (SIDA), le virus de la vaccine, le coronavirus, le papillomavirus, le parvovirus, le virus de la fièvre catarrhale du mouton, le virus de la Dengue, le virus Ebola, ou encore la grippe, la variole, la rougeole, la rubéole, la varicelle, l'hépatite A, B, C, D ou E, la mononucléose, la fièvre jaune, l'encéphalite ou l'herpès.

13. Utilisation d'une composition pharmaceutique selon la revendication 9, pour la fabrication d'un médicament anticancéreux destiné à prévenir et/ou à traiter un sujet atteint d'un cancer, tel que le cancer du poumon, de l'utérus, du sein ou de l'ovaire, le cancer colorectal, la leucémie ou un sujet atteint d'une tumeur de la prostate, de la vessie, de la peau, du cerveau, de la gorge.

14. Méthode de prévention et/ou de traitement d'une maladie chez une plante au moyen d'un produit phytopharmaceutique comprenant une Mutactimycine ou une Aldgamycine telle que définie aux revendications 1 à 8.
